# EUROPEAN PATENT SPECIFICATION

(11) **EP 3 363 445 B1**
(45) Date of publication and mention of the grant of the patent: **09.12.2020**
(21) Application number: 16855565.4
(22) Date of filing: 17.10.2016
(51) Int. Cl.: A61K 35/644, A61P 25/28

(54) **METHOD FOR PRODUCING ROYAL JELLY FRACTION ANT ITS USE FOR TREATING ALZHEIMER'S DISEASE**
VERFAHREN ZUR HERSTELLUNG EINER GELEE-ROYALE-FRAKTION UND DEREN VERWENDUNG ZUR BEHANDLUNG DER ALZHEIMER-KRANKHEIT
PROCÉDÉ DE PRODUCTION DE FRACTION DE GELÉE ROYALE ET SON UTILISATION POUR LE TRAITEMENT DE LA MALADIE D'ALZEIMER

(30) Priority: 16.10.2015 JP 2015204320
(43) Date of publication of application: 22.08.2018
(73) Proprietor: Tohoku University, Sendai-shi, Miyagi 980-8577 (JP); Japan Royal Jelly Co., Ltd., Tokyo 163-0636 (JP)
(72) Inventor: YAMAKUNI, Toru, Sendai-shi Miyagi 980-8577 (JP); KAWAHATA, Ichiro, Sendai-shi Miyagi 980-8577 (JP); YAMAGUCHI, Kikuji, Tokyo 163-0636 (JP); MURATA, Kiyoshi, Tokyo 163-0636 (JP)
(74) Representative: Hoffmann Eitle
(86) International application number: PCT/JP2016/080718
(87) International publication number: WO 2017/065314

(56) References cited:
- WO-A1-2008/149838
- WO-A1-2009/154197
- CN-A- 102 260 728
- CN-A- 104 415 025
- JP-A- 2008 228 727
- JP-A- 2009 029 772

## Description

### TECHNICAL FIELD

The present invention is related to methods for preparing royal jelly fractions.

### BACKGROUND ART

Dementia includes such as dementia of Alzheimer's type, cerebrovascular dementia, Lewy body dementia, and frontotemporal lobar degeneration, and it is said that 60 - 70% of dementia is a dementia of Alzheimer's type. Dementia of Alzheimer's type includes Alzheimer's presenile dementia (AD, also referred to as early-onset Alzheimer's disease) and senile dementia of the Alzheimer type (SDAT). In general, the cut-off age of the onset of Alzheimer's-type dementia (ATD) is 65 years old, and this is divided into the onset of Alzheimer's disease (AD) at age under 65 years and the onset of senile dementia of the Alzheimer type (SDAT) at age 65 years and older. In AD, it is known that the hippocampus and entorhinal cortex which govern the memory are those that are affected from the very early stage.

In particular, of memory impairment, episodic memory is likely to be impaired in AD. For episodic memory, hippocampus and entorhinal cortex play an important role. Entorhinal cortex has a role to bridge the hippocampus and the cerebral neocortex, and memory is stored in the cerebral cortex which is passed from the hippocampus through the entorhinal cortex. However, as the entorhinal cortex is impaired from the early stage of AD, memory cannot be stored in the cerebral cortex.

Long-term potentiation (LTP) in the hippocampus is believed to be one of the important mechanism in memorizing and learning. It has recently been reported that, in cognitive impairment such as AD, various factors cause synaptic plasticity impairment, causing the suppression of LTP which results in memory impairment. One of the important molecular target of this synaptic plasticity is the phosphorylation of the cAMP response element binding protein (CREB) and the subsequent CRE-dependent transcription activity. Soluble amyloid-β (Aβ) oligomers are believed to be the cause of the initial cognitive impairment of AD. These Aβ oligomers suppress the CRE-dependent transcription activity that is closely associated to memory formation in hippocampal neurons.

cAMP/PKA/ERK/CREB-dependent signaling pathway has been demonstrated to be closely associated to memory and learning ability of the brain. Recently, in addition to the degeneration and loss of neurons due to the accumulation of amyloid β protein (Aβ) in the brain, synaptic plasticity impairment without neuronal cell death caused by soluble Aβ has been understood to be one of the cause of memory impairment in Alzheimer's disease (AD). For example, long-term potentiation (LTP) of neurotransmission in glutamate synapses of the hippocampus which is believed to be one of the cellular mechanism of learning and memory formation is considered to be a representative example of synaptic plasticity. Aβ is known to inhibit the PKA/CREB signaling and to suppress the occurrence of this LTP. In other words, it is believed that a food containing physiological active substances which improve or suppress the inhibition of the signaling caused by such Aβ is considered to be useful for the improvement and prevention of AD.

In the cerebrum of AD patients, asenile plaques and neurofibrillary tangles appear. In the cerebral cortex, first the senile plaques appear, and next neurofibrillary tangles appear. For this reason, initially, the cause of AD was believed to be senile plaques. However, based on the results of clinical studies that even after the disappearance of senile plaques, memory impairment was unable to be improved, it was concluded that the senile plaques were the outcome of AD rather than the cause. More recently, soluble amyloid β (Aβ) oligomers are believed to be the cause memory impairment manifesting at the early stage of AD.

Recently, a system degrading Aβ oligomer which is a causative agent of AD was found in the hippocampus. More specifically, an Aβ oligomer accumulation suppression system consisting of hippocampal neurons making Aβ oligomer degrading enzyme (neprilysin) and hippocampal neurons making a factor which strengthens the action of neprilysin (somatostatin) was discovered.

Aβ is a molecule consisting of 42 amino acids, and a structural change takes place due to some cause, and two of this are gathered together to form an Aβ oligomer. This Aβ oligomer has been demonstrated to have a toxicity which damages the neurons in the hippocampus, which causes memory impairment and cell death. Furthermore, an activity to induce neurofibrillary tangles in the hippocampus and cerebral cortex is also present. Moreover, the action of this degrading enzyme and the expression of this activity enhancer are attenuated by aging and AD. Therefore, the development of functional foods that can recover and strengthen the system of Aβ oligomer degradation in the hippocampus has been awaited.

Freeze royal jelly is known to have a CRE-dependent transcription promoting action on a neuron model (PC 12-D) (Patent Document 1). However, actions on the cells constituting the hippocampus that governs *in vivo* memory have not been confirmed. Also, what fraction of the royal jelly is more effective is not known.

JP 2008 228727 A relates to a material containing royal jelly-decomposing enzyme that is prepared by a method including the following processes from the 2-3 day old queen bee larvae of Apis mellifera: (a) a process of centrifugal treatment of the suspension of body tissues of the queen bee larvae at ≤6°C so as to separate to an upper layer of white solid form, a middle layer of solution and a lower layer of precipitate; and (b) a process of recovering the middle layer as the material containing royal jelly-decomposing enzyme. CN 102 260 728 A relates to a royal jelly polypeptide and application thereof. According to the document, intestinal tract enzyme is extracted from a queen bee larva in vivo, and an in vivo enzymolysis environment of queen bee is simulated to carry out enzymolysis on royal jelly so as to obtain 1-3kDa of royal jelly polypeptide. The royal jelly polypeptide can be used for preparing an A beta inhibitor.

JP 2009 029772 A relates to an enzyme-treated royal jelly characterized in that it is prepared by adding an extractive solvent, e.g. a hydrous ethanol, to at least one selected from a raw royal jelly and a dried royal jelly, to separate a soluble fraction, and reacting a fraction insoluble to the above extractive solvent with a neutral protease, e.g. thermolysin.

CN 104 415 025 A relates to a composition for delaying progress of a degenerative disease. The composition comprises royal jelly acid or a royal jelly acid derivative and gamma-aminobutyric acid or a gamma-aminobutyric acid derivative, and the ratio in parts by weight is (0.1-10): (0-100).

WO 2008/149838 A1 relates to a fractionation product of royal jelly, which comprises 1 part by mass of 10-hydroxy-2-decenoic acid and less than 0.05 part by mass of a protein derived from royal jelly, which is said to have a useful physiological activity of royal jelly, and which is said to have such a property that no allergenicity against the fractionation product is detected by the passive cutaneous anaphylaxis on a rat using a serum from a mouse immunized with such an amount of the fractionation product that 45 µg of 10-hydroxy-2-decenoic acid is contained in the fractionation product; a method for producing the fractionation product; and use of the fractionation product.

### PRIOR ART DOCUMENTS

### PATENT DOCUMENTS

Patent Document 1: WO2009/154197 A

### SUMMARY OF INVENTION

### Technical Problem

The present invention provides a method for preparing royal jelly fraction having a higher CRE-dependent transcription promoting action compared to the prior arts.

### SOLUTION TO PROBLEM

The present invention relates to the following:
[1] a method for preparing a royal jelly fraction comprising: eluting a royal jelly with water to obtain a residue; eluting the obtained residue with 50% ethanol to obtain a residue, and eluting the obtained residue with 99.5% ethanol to obtain an elution fraction;
[2] a composition comprising royal jelly for use in treating or preventing cognitive impairment or Alzheimer's disease, wherein the royal jelly is a royal jelly fraction obtained by the method of [1].

### ADVANTAGEOUS EFFECTS OF THE INVENTION

The Royal Jelly fractions of the present invention are useful in the prevention and/or treatment of Alzheimer's disease.

### BRIEF DESCRIPTION OF THE DRAWINGS

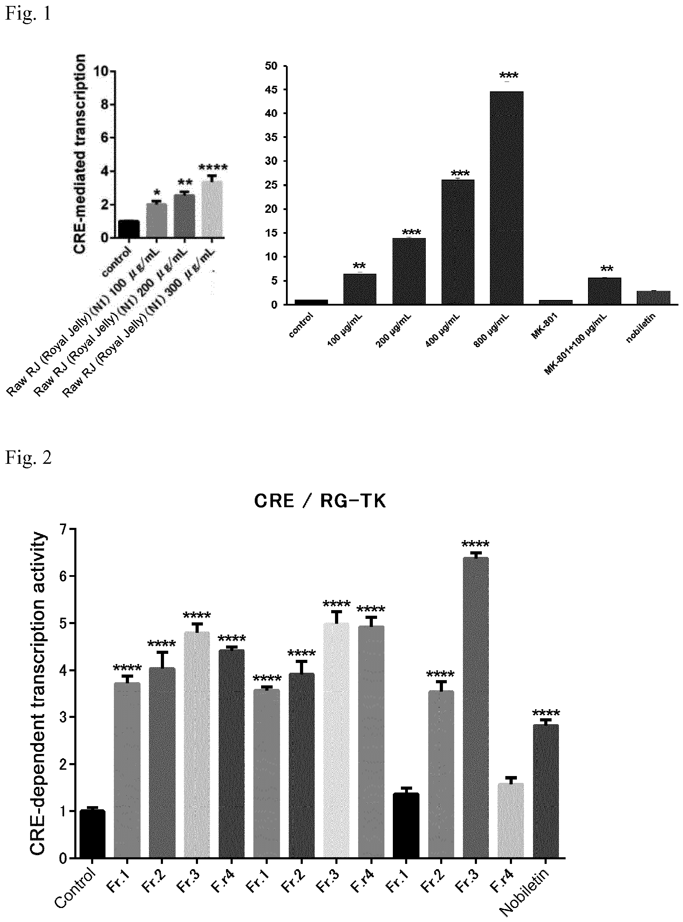

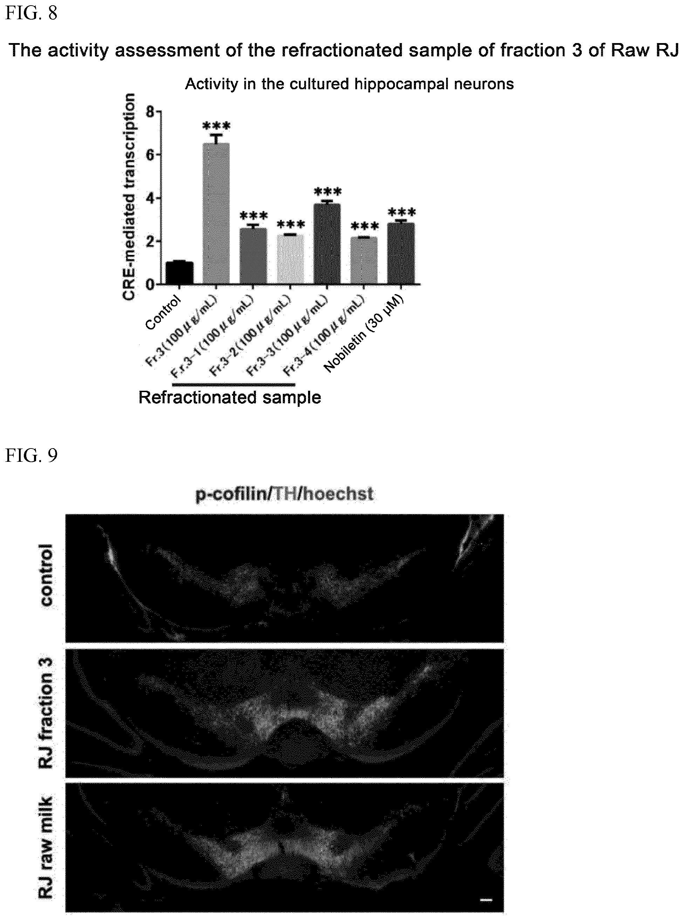

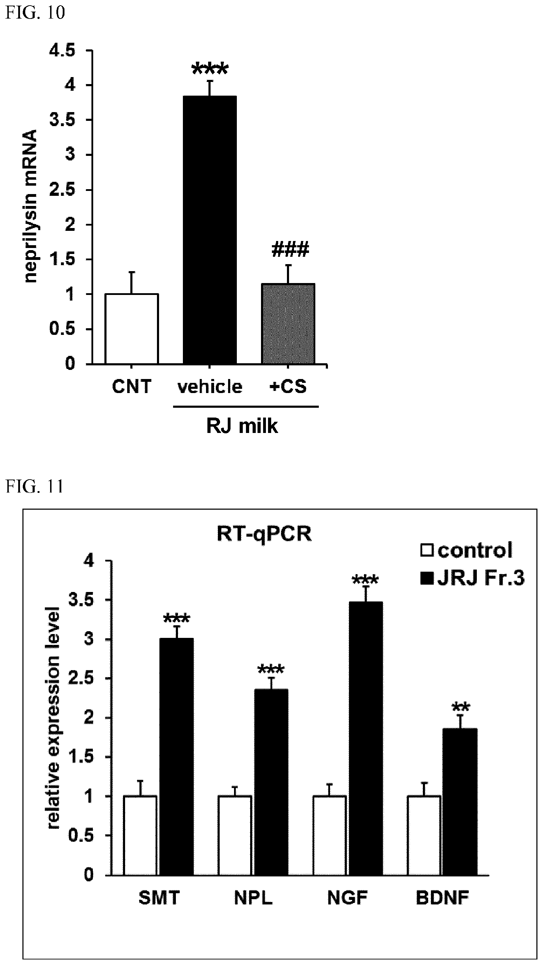

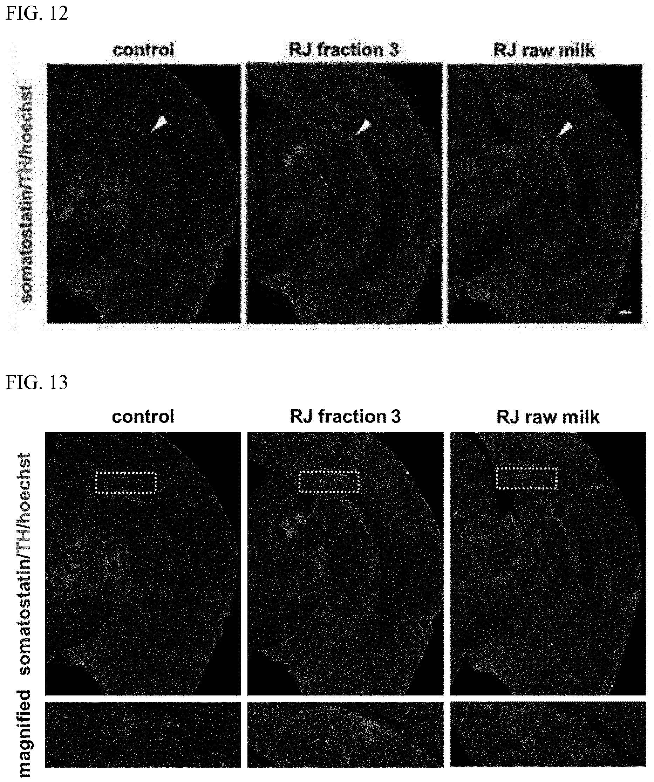

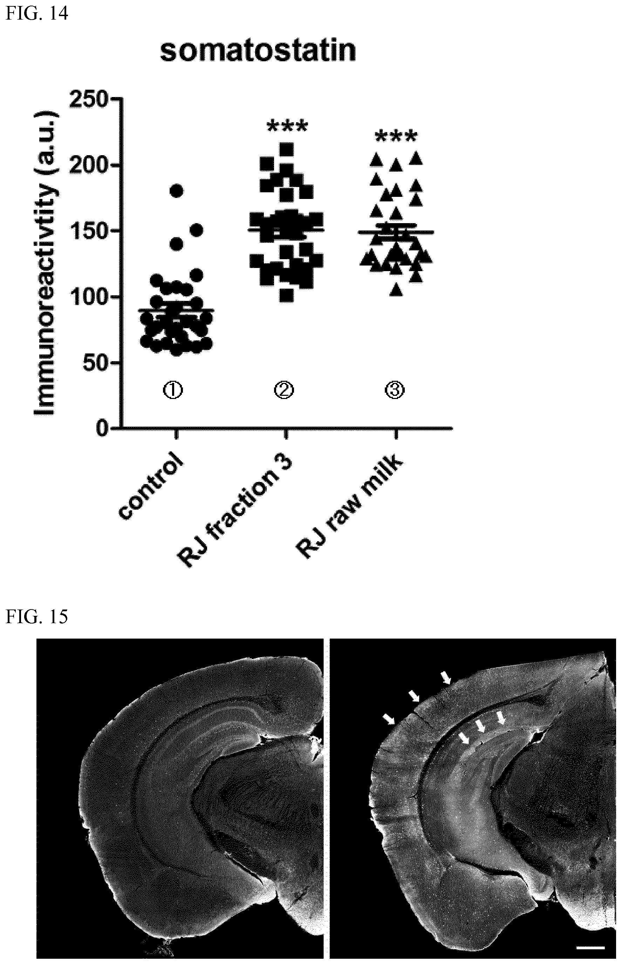

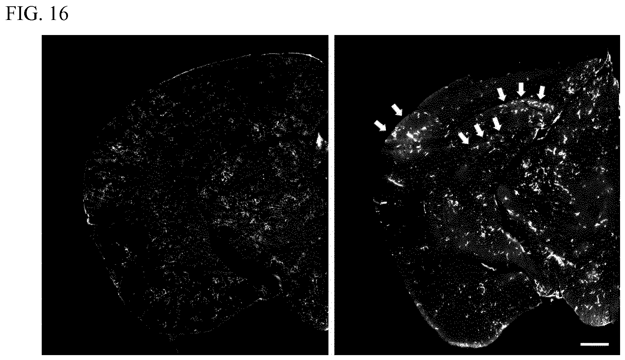

FIG. 1: A sample in which raw royal jelly dissolved in DMSO shows a strong CRE-dependent transcription promoting activity as compared to the PBS (-) dissolved raw royal jelly. The left shows a PBS (-) dissolved raw royal jelly and the right shows a DMSO dissolved raw royal jelly. The values are expressed as mean ± SEM (n=4). Compared to the control, *** is p <0.001, ** is p <0.01. MK-801 is 10 µM and nobiletin is a 30 µM treatment.
FIG. 2: Transcription promoting activity is also confirmed in fractions other than the water-soluble fraction of raw royal jelly (Fr. 2, 3, 4). For all pairs, a one-way analysis of variance followed by post Tukey's test is performed. Each royal jelly fraction is treated at 100 µg/ml. Nobiletin is treated at 30µM. The values are expressed as mean ± SEM (n=4) in all experiments. Compared to the control, **** is p <0.0001, ** is p <0.01.

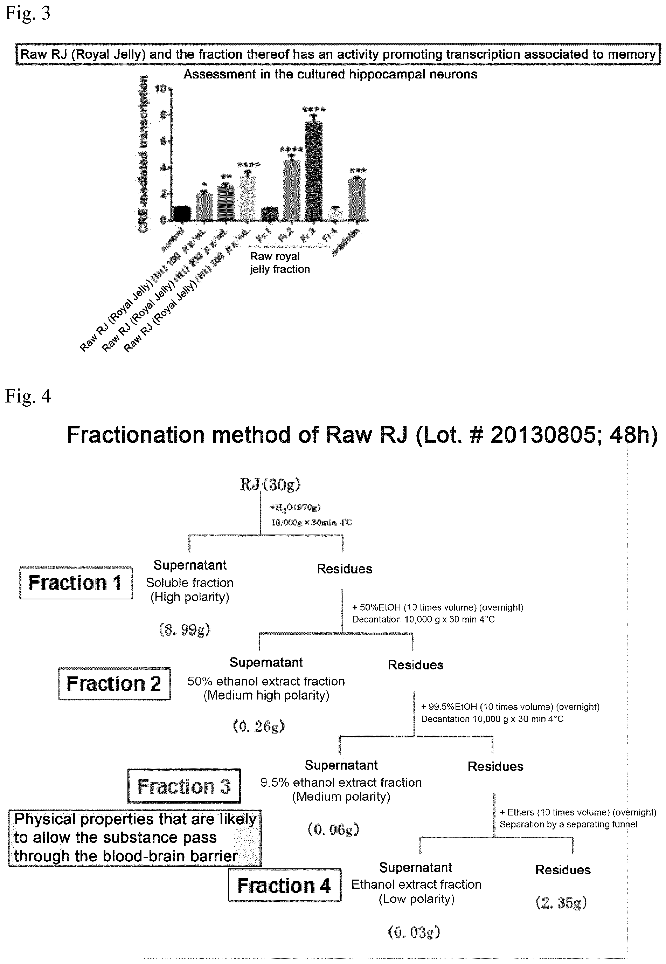

FIG. 3: Figure 3 shows a transcription promoting activity associated with memory in raw royal jelly and the fraction thereof. For all pairs, a one-way analysis of variance followed by post Tukey's test is performed. The values are expressed as mean ± SEM (n=4). Compared to the control, **** is p <0.0001, * is p <0.05. Raw royal jelly fraction is 100 µg/ml and nobiletin is treated at 30 µM.
FIG. 4: Figure 4 shows a fractionation method of a raw royal jelly. The water-soluble fraction is dissolved in PBS (-) and the others are dissolved in DMSO to evaluate the activity.

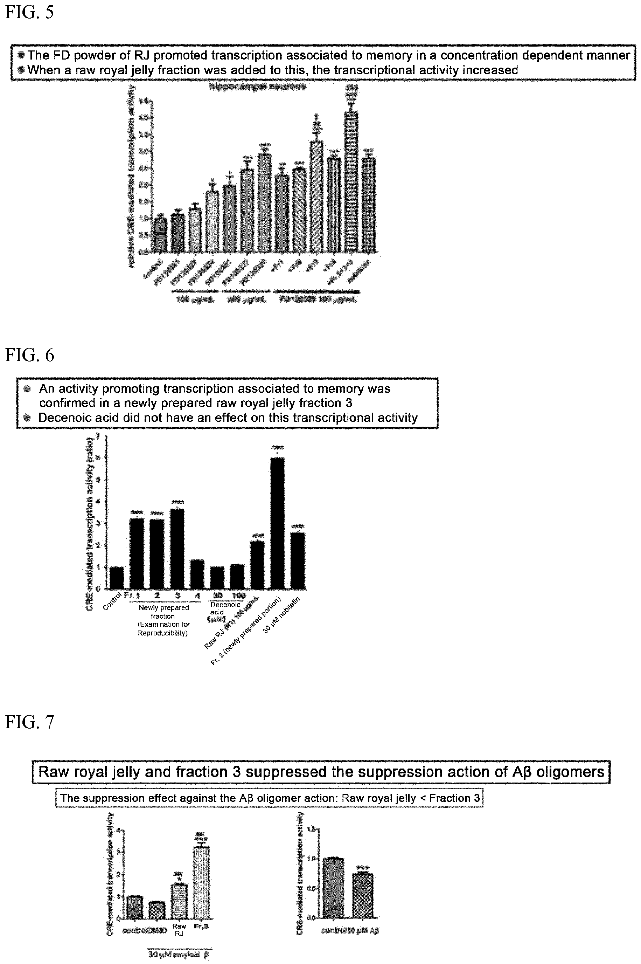

FIG. 5: Figure 5 shows a transcription promoting activity associated with memory in the freeze dry powder (FD powder) royal jelly and each fraction. For all pairs, a one-way analysis of variance followed by post Tukey's test is performed. The values are expressed as mean ± SEM (n=4). Compared to the control, *** is p <0.001, ** is p <0.01, and * is p <0.05. Compared to FD120329 (freeze-dried powder 100 µg/ml and each fraction 100 µg/ml), ## is p <0.01, and ### is p <0.001. Compared to nobiletin (30 µM), $$$ is p <0.001, and $ is p <0.05.
FIG. 6: Figure 6 shows a transcription promoting activity associated with memory in the royal jelly fraction 3. For all pairs, a one-way analysis of variance followed by post Tukey's test is performed. The values are expressed as mean ± SEM (n=4). Compared to the control, **** is p <0.0001, * is p <0.05. Fr. 1 - 4 are 100 µg/ml, and nobiletin is treated at 30 µM.
FIG. 7: The left side of Figure 7 shows a suppressive effect on the action of Aβ oligomers in raw royal jelly and fraction 3. For all pairs, a one-way analysis of variance followed by post Tukey's test is performed. Compared to 30 µM amyloid β-treated cells, *** is p <0.001, * is p <0.05. Raw royal jelly fraction is 100 µg/ml, and nobiletin is treated at 30 µM. The right side of Figure 7 shows how Aβ affects CRE-dependent transcription activity. Student's t-test is used. Compared to the control, *** is p <0.001.
FIG. 8: Figure 8 shows the effect of fraction 3 of the royal jelly on the CRE-dependent transcription activity in primary cultured hippocampal neurons. For all pairs, a one-way analysis of variance followed by post Tukey's test is performed. The values are expressed as mean ± SEM (n=4). Compared to the control, *** is p <0.001.
FIG. 9: Figure 9 shows the expression levels of tyrosine hydroxylase (TH) which is a rate-determining enzyme of dopamine synthesis and phosphorylated cofilin resulting from the raw royal jelly and fraction 3 in the dopamine nerve of an adult mouse midbrain (substantia nigra, ventral tegmental area). After a one week oral administration of 8 mg/kg of the third fraction of royal jelly or 0.5 g/kg of the raw royal jelly, perfusion fixation is carried out to prepare thin sections, and the midbrain tissue is stained. An increase in the expression of phosphorylated cofilin levels and tyrosine hydroxylase are observed. The scale bar is 300 µm.
FIG. 10: Figure 10 shows the effect of raw royal jelly (100 µg/ml) on the mRNA expression level of neprilysin in the cultured hippocampal neurons. Each mRNA level is normalized by β-actin. The values are expressed as mean ± SEM. Compared to the control (empty column), *** is p <0.001. Compared to the vehicle (packed column), ### is p <0.001. n is 3. CNT is a control, and CS is a 10 µM cyclosomatostatin.
FIG. 11: The effect of royal jelly fraction 3 on mRNA expression levels of somatostatin, neprilysin, NGF, and BDNF in cultured hippocampal neurons. Each mRNA level is normalized by β-actin. The values are expressed as mean ± SEM. Compared to the control, *** is p <0.001, ** is p <0.01. n is 3. SMT is a somatostatin and NPL is a neprilysin.
FIG. 12: Figure 12 shows the enhanced expression of somatostatin resulting from raw royal jelly and royal jelly fraction 3 in adult mouse hippocampal neurons. The scale bar is 200 µm. The stained portion pointed by the arrow indicates the somatostatin expression.
FIG. 13: Figure 13 shows the enhanced expression of somatostatin by raw royal jelly and royal jelly fraction 3 in adult mouse hippocampal neurons. The bottom column is an enlarged view of the portion encircled by the dotted lines in the upper column.
FIG. 14: Figure 14 shows the enhanced expression of somatostatin resulting from raw royal jelly and royal jelly fraction 3 in adult mouse hippocampal neurons. 1-3 corresponds to the control, royal jelly fraction 3, and raw royal jelly, respectively, in the enlarged view of FIG. 13. Somatostatin-positive neurons in each treatment group of the immunohistochemical staining results are individually analyzed, and the somatostatin expression levels are shown on the vertical axis. 1. Black circles indicate the analysis results of 30 cells of the control group; 2. Black squares indicate the analysis results of 32 cells of the treatment group of royal jelly fraction 3; and 3. Black triangles indicate the analysis results of 30 cells of the raw royal jelly treatment group. Royal jelly fraction 3, raw royal jelly treated group are both *** p <0.001 versus control (A significance test using Tukey's multiple comparison test).
FIG. 15: Figure 15 shows results of somatostatin expression enhancing effect resulting from the oral administration of raw RJ (Royal Jelly) (RJ milk) in the hippocampus of aged mice. A one week oral administration of 0.5 g/kg raw RJ in 17 months old C57BL6 strain aged mice enhanced the expression level of somatostatin in the cerebral cortex and hippocampus (arrows) in the immunohistochemical analysis of the brain tissue sections. The scale bar is 200 µm.
FIG. 16: Expression enhancing effect of neprilysin resulting from the oral administration of raw RJ in the hippocampus of aged mice. A one week oral administration of 0.5 g/kg of raw RJ in the same mice enhanced the expression level of neprilysin in the cerebral cortex and hippocampus (arrows). The scale bar is 200 µm.

Fractions of royal jelly obtained by the method of preparation of the present invention may be formulated in pharmaceuticals, quasi drugs, food, or the like, for improving cognitive impairment.

Forms of the pharmaceuticals include such as tablets, capsules, granules, and syrup. These pharmaceuticals can be prepared by using additives in conventional pharmaceutical preparation.

The dosage of the cognitive impairment improving agent of the present invention is not particularly limited; however, in case of oral administration, it is 10 - 60 mg/kg of body weight per day, preferably 20 mg/kg of body weight per day, and in case of parenteral administration, it is 2 - 6 mg/kg of body weight per day, preferably 2 - 3 mg/kg of body weight per day. The above-mentioned dosage can be administered in a single dose or divided into 2 - 3 times a day, and depending on the age, disease state, and symptoms, it may be appropriately increased or decreased.

Specific examples of the additives include such as lactose, dextrin, sucrose, mannitol, corn starch, sorbitol, crystalline cellulose, and polyvinylpyrrolidone, and these may be used alone or in combination as appropriate. According to the description of the Japanese Pharmacopoeia, these pharmaceuticals can be prepared by a method which is suitable for the form of each pharmaceutical. Moreover, flavoring agents, coloring agents, sweetening agents, and the like, may be appropriately used. The content of these additives can be appropriately chosen by those skilled in the art.

Forms of quasi drugs include such as tablets, capsules, granules, jellies, and drinks. These quasi drugs can be prepared using additives of the conventional quasi-drug preparation. Moreover, these quasi drugs, for example, can also contain other active ingredients such as vitamins. Furthermore, additives such as sweetening agents, flavoring agents, coloring agents, and antioxidants may be used alone or in combination as appropriate. These quasi drugs can be prepared by methods well-known to those skilled in the art.

The forms of food include such as noodles, pastas, granules, tablet candies, jellies and liquids (beverages). These foods can be prepared by appropriately using a variety of food materials. Specific examples of food ingredients include such as rice, wheat, corn, potato, sweet potato, soybean flour, seaweed powder, syrup, lactose, glucose, fructose, sucrose, and mannitol, and these may be used alone or in combination as appropriate. If necessary, it can have a desired shape by using such as water, if necessary. Furthermore, flavoring agents, coloring agents, sweetening agents, cooking oils, and vitamins may be added as appropriate.

Furthermore, the present invention is related to a composition according to claim 1 for use in a treatment and/or preventive method of cognitive impairment comprising administering royal jelly fractions of the present invention to a subject in need for improving cognitive impairment, and more specifically, it is related to.

### DESCRIPTION OF EMBODIMENTS

Hereinbelow, the present invention is specifically explained in the Examples; however, this is merely an illustration of the representative examples, and the present invention is not limited to these examples.

### EXAMPLES

A part of the brain called hippocampus is essential for the executive function of the brain storing personal daily events/activities and words. At the initial stage of Alzheimer's disease which is the biggest cause disease of dementia, it is known that the dysfunction of neurons in this hippocampus occurs.
The action of the royal jelly was examined using the transcriptional activity associated with memory formation in the hippocampus as an indicator. From the hippocampus of mouse brain, neurons were taken out and cultured for 2 weeks, and the subsequent addition of fresh raw royal jelly to the culture solution resulted in the response of the hippocampal neurons and the transcriptional activity was increased. Furthermore, it was observed that treating neurons with a concentrated active ingredient resulted in a stronger response. From this study, royal jelly has been shown to have an action to enhance the activity of hippocampal neurons associated with memory.

### Fractionation Methods

1. An open column (diameter 25 mm × total length 550 mm) was packed with Diaion (HP-20, 20g) and then equilibrated by continuous water flow for 2 - 3 hours.
2. Supernatant (Fraction 3) was concentrated by an evaporator until 1 ml - 2 ml, and the total volume was filled into the column.
3. Water (100ml) was gradually added to the column, eluted, and fractions were obtained.
4. 50% ethanol (100ml) was gradually added to the column, eluted, and fractions were obtained.
5. 99.5% ethanol (100ml) was gradually added to the column, eluted, and fractions were obtained.
6. Methanol (100ml) was gradually added, eluted, and fractions were obtained.
7. The solvents of the fractions obtained in the above 2 - 5 were distilled away under reduced pressure, and the yield was measured.

The following shows the yield of each fraction.

**[Table 1]**

| **Yield of each fraction** | | | |
|---|---|---|---|
| Sample Name | | (g) | (Dry Weight%) |
| H₂O Eluted fraction | (Fr.3-1) | 0.02031 | (20.5%) |
| 50%EtOH Eluted fraction | (Fr.3-2) | 0.00866 | (8.8%) |
| 99.5%EtOH Eluted fraction | (Fr.3-3) | 0.04954 | (50.1%) |
| MeOH Eluted fraction | (Fr.3-4) | 0.02036 | (20.6%) |
| Total | | 0.09887 | (100%) |

The assessment results of the hippocampal neuron system cultured for 14 days

Samples prepared by refractionating fraction 3 of the raw RJ (Fr.3-1, Fr.3-2, Fr.3-3, Fr.3-4) were examined on whether they promote the activity associated with memory formation in the hippocampus. In all Fr. 3-1, Fr. 3-2, Fr. 3-3, and Fr. 3-4, a promotion of activity was observed with the treatment concentration of 100 µg/mL. However, when the yield of each fraction is taken into consideration, it is calculated that Fr. 3-3 has the strongest activity (FIG. 8).

The royal jelly and each fraction were assessed in the hippocampal neuron system cultured for 14 days.
1. Concentration-dependent CRE-dependent transcription promoting activity was observed in fresh raw royal jelly collected after 48 hours.
2. CRE-dependent transcription promoting activity was observed in raw royal jelly in hippocampal neuron culture system.
3. In 100 µg/ml of raw royal jelly fractions 2 and 3, in particular, in fraction 3, a very strong CRE-dependent transcription promoting activity was observed. Moreover, the transcription promoting effect is more potent than that of 30 µM nobiletin and it was 7 times of the control group.
4. When raw royal jelly fraction was added to the freeze dried powder of royal jelly, transcriptional activity was increased.
5. Raw royal jelly and fraction 3 suppressed the suppression action of Aβ oligomers.

The expression levels of tyrosine hydroxylase (TH) which is a rate-determining enzyme of dopamine synthesis and phosphorylated cofilin resulting from the raw royal jelly and fraction 3 in the dopamine nerve of adult mouse midbrain (substantia nigra, ventral tegmental area), were examined. After a one week oral administration of 8 mg/kg of the third fraction of royal jelly or 0.5 g/kg of the raw royal jelly, perfusion fixation was carried out to prepare thin sections, and the midbrain tissue was stained. An increase in the expression of phosphorylated cofilin levels and tyrosine hydroxylase was observed (Fig. 9).

In hippocampal neurons expressing an Aβ oligomer degrading enzyme (neprilysin) and hippocampal neurons expressing a factor which strengthens the action of neprilysin (somatostatin), raw royal jelly and the fraction thereof enhanced the expression of neprilysin and somatostatin. Then, the royal jelly can recover and strengthen the degradation system of Aβ oligomers in the hippocampus.

Raw royal jelly increased the neprilysin mRNA levels in a somatostatin receptor-dependent manner. Hippocampal neuron culture system was used as a system to assess the activity of somatostatin-neprilysin system. Cyclosomatostatin (CS) which is a non-selective somatostatin receptor antagonist suppressed the enhanced expression of neprilysin mRNA by RJ (Fig. 10).

Raw royal jelly fraction 3, neprilysin, somatostatin which is its activity enhancer increased the mRNA levels of NGF and BDNF (Fig. 11).

The action of the raw royal jelly and fraction 3 was examined *in vivo.* After a one week oral administration of 8 mg/kg of royal jelly fraction 3 or 0.5 g/kg of the raw royal jelly, perfusion fixation was carried out to prepare thin sections, and the hippocampal tissue was stained. As a result of this, an increase in immune reaction of somatostatin and positive reaction was observed (Figs. 12, 13, and 14).

The effect of raw royal jelly on the expression of neprilysin and somatostatin which is an enhancer thereof in the hippocampus of aged mice was immunohistochemically analyzed. Raw royal jelly (500 mg/kg of saline extract) was orally administered for 7 days to C57BL/6N mice (17 months old, male). As a result of this, in the hippocampus of aged mice, an enhanced expression of neprilysin and somatostatin caused by raw royal jelly was observed (Figs. 15 and 16).

## Claims

1. A method for preparing a royal jelly fraction comprising:
eluting a royal jelly with water to obtain a residue;
eluting the obtained residue with 50% ethanol to obtain a residue, and
eluting the obtained residue with 99.5% ethanol to obtain an elution fraction.

2. A composition comprising royal jelly for use in treating or preventing cognitive impairment or Alzheimer's disease, wherein the royal jelly is a royal jelly fraction obtained by the method of claim 1.

## Patentansprüche

1. Verfahren zur Herstellung einer Gelee Royale Fraktion, umfassend:
Eluieren eines Gelee Royale mit Wasser, um einen Rückstand zu erhalten;
Eluieren des erhaltenen Rückstands mit 50% Ethanol, um einen Rückstand zu erhalten, und
Eluieren des erhaltenen Rückstands mit 99,5% Ethanol, um eine Elutionsfraktion zu erhalten.

2. Zusammensetzung, umfassend Gelee Royale zur Verwendung bei der Behandlung oder Vorbeugung von kognitiver Beeinträchtigung oder Alzheimer, wobei das Gelee Royale eine Gelee Royale Fraktion ist, die durch das Verfahren gemäß Anspruch 1 erhalten wird.

## Revendications

1. Procédé de préparation d'une fraction de gelée royale comprenant les étapes consistant à :
éluer une gelée royale avec de l'eau pour obtenir un résidu ;
éluer le résidu obtenu avec de l'éthanol à 50 % pour obtenir un résidu, et
éluer le résidu obtenu avec de l'éthanol à 99,5 % pour obtenir une fraction d'élution.

2. Composition comprenant de la gelée royale destinée à être utilisée dans le traitement ou la prévention de troubles cognitifs ou de la maladie d'Alzheimer, dans laquelle la gelée royale est une fraction de gelée royale obtenue par le procédé selon la revendication 1.
